(19)

Europäisches
Patentamt
European
Patent Office
Office européen
des brevets

(11) **EP 4 699 649 A1**

(12) **EUROPEAN PATENT APPLICATION**

(43) Date of publication:
**25.02.2026 Bulletin 2026/09**

(21) Application number: **25196762.6**

(22) Date of filing: **19.08.2025**

(51) International Patent Classification (IPC):
***A61N 5/10*** (2006.01)

(52) Cooperative Patent Classification (CPC):
**A61N 5/1075;** A61N 5/1069; A61N 5/1081;
A61N 2005/1076

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **20.08.2024 US 202418809563**

(71) Applicant: **Siemens Healthineers International AG
6312 Steinhausen (CH)**

(72) Inventors:
• **Kieselmann, Jennifer
5400 Baden (CH)**
• **Thieme-Marti, Stefan
5210 Windisch (CH)**

(74) Representative: **Mathisen & Macara LLP
Charta House
30-38 Church Street
Staines-upon-Thames TW18 4EP (GB)**

(54) **NUMERICAL CALIBRATION APPROACH FOR TREATMENT COUCH CALIBRATION**

(57) Example methods (400) and systems (180) for treatment couch calibration are described. In one example, a computer system (180) may obtain (411, 412) multiple candidate datasets and multiple first couch models. Based on the multiple candidate datasets and the multiple first couch models, the computer system (180) may perform (420) numerical calibration to generate (421) multiple second couch models. Based on the multiple first couch models and the multiple second couch models, the computer system (180) may determine (430) metric data associated with the multiple candidate datasets. The computer system (180) may select, from the multiple candidate datasets, a first dataset based on the metric data. The first dataset may be selected for use by a controller associated with a treatment couch (131) to cause the treatment couch (131) to move towards each of the multiple first calibration poses during treatment couch calibration.

| Degrees of Freedom (DoF)* | | |
|---|---|---|
| X | Lateral | 321 |
| Y | Longitudinal | 322 |
| Z | Vertical | 323 |
| $\theta_X$ | Pitch | 324 |
| $\theta_Y$ | Roll | 325 |
| $\theta_Z$ | Yaw | 326 |

*\* Examples: 3DoF, 4DoF, 6DoF, etc.*

**FIG. 3**

**Description**

CROSS-REFERENCE TO RELATED APPLICATION

The present application (Attorney Case No. 09678) is related in subject matter to European Patent Application entitled "Kinematic Calibration of a Treatment Couch for Radiation Therapy" (Attorney Case No. 09679), with priority date 20 August 2024, and priority application number 18/809,573, which is incorporated herein by reference. The two applications are being filed on the same day.

BACKGROUND

Radiation therapy is a widely used cancer treatment modality that uses high-energy radiation to reduce or eliminate cancerous tumors. In practice, applied radiation does not inherently discriminate between a tumor and proximal healthy structures within a patient, such as organs, etc. Ideally, the objective is to deliver a lethal or curative radiation dose to the tumor, while maintaining an acceptable dose level in the proximal healthy structures. In practice, treatment couch calibration is performed to ensure precise and accurate delivery of radiation doses to the patient. Calibration helps to ensure that the treatment couch is positioned accurately during radiation therapy, such as relative to a linear accelerator (LINAC) that delivers radiation doses. Any inaccuracy associated with the positioning of the treatment couch, as well as that of the patient, may affect the accuracy and effectiveness of radiation treatment delivery.

BRIEF DESCRIPTION OF DRAWINGS

FIG. 1 is a schematic diagram illustrating an example radiation therapy system that includes a treatment delivery system;

FIG. 2 is a schematic diagram illustrating a perspective view of the treatment delivery machine shown in the example in FIG. 1;

FIG. 3 is a schematic diagram illustrating an example detailed configuration of a couch positioning assembly shown in the example in FIGs. 1-2.

FIG. 4 is a flowchart of an example process for a first computer system to perform a numerical calibration approach for calibration dataset generation and a second computer system to perform treatment couch calibration;

FIG. 5 is a schematic diagram illustrating example kinematic modelling for a treatment couch;

FIG. 6 is a flowchart of an example detailed process for a first computer system to perform a numerical calibration approach for calibration dataset generation;

FIG. 7 is a diagram illustrating example numerical calibration approach for calibration dataset generation according to the example in FIG. 6;

FIG. 8A is a first table illustrating example metric data associated with multiple candidate datasets;

FIG. 8B is a second table illustrating example metric data associated with multiple candidate dataset sizes ($N$);

FIG. 9A are example boxplots displaying average and maximum deviation data for linear and rotational components for a dataset size of $N = 50$ poses;

FIG. 9B are example boxplots displaying average and maximum deviation data for linear and rotational components for a dataset size of $N = 100$ poses;

FIG. 10A are example boxplots displaying average and maximum deviation data for linear and rotational components for a dataset size of $N = 150$ poses; and

FIG. 10B are example boxplots displaying average and maximum deviation data for linear and rotational components for a dataset size of $N$ = 200 poses.

SUMMARY

According to a first aspect of the invention, there is provided a method for a computer system to perform a numerical calibration approach for treatment couch calibration, the method as defined by claim 1.

According to a second aspect of the invention, there is provided a computer system as defined by claim 6.

According to a third aspect of the invention, there is provided a radiation therapy system, as defined by claim 11.

Optional features are defined by the dependent claims.

According to examples of the present disclosure, treatment couch calibration may be performed in an improved manner using a numerical calibration approach. In practice, examples of the present disclosure may be implemented to alleviate one or more challenges associated with treatment couch calibration, particularly the selection or generation of a suitable dataset of calibration poses (also known as calibration measurement positions). Instead of using grid-based sampling that involves a large number of calibration measurements, a numerical approach may be implemented to perform a metric-based evaluation of multiple candidate datasets. In practice, any improvement in couch calibration accuracy may lead to more accurate and safer positioning of a patient during radiation treatment. As used herein, the term "numerical calibration" may refer generally to computational procedure(s) or algorithm(s) for generating or selecting a dataset of calibration poses for use in treatment couch calibration.

(a) Dataset selection

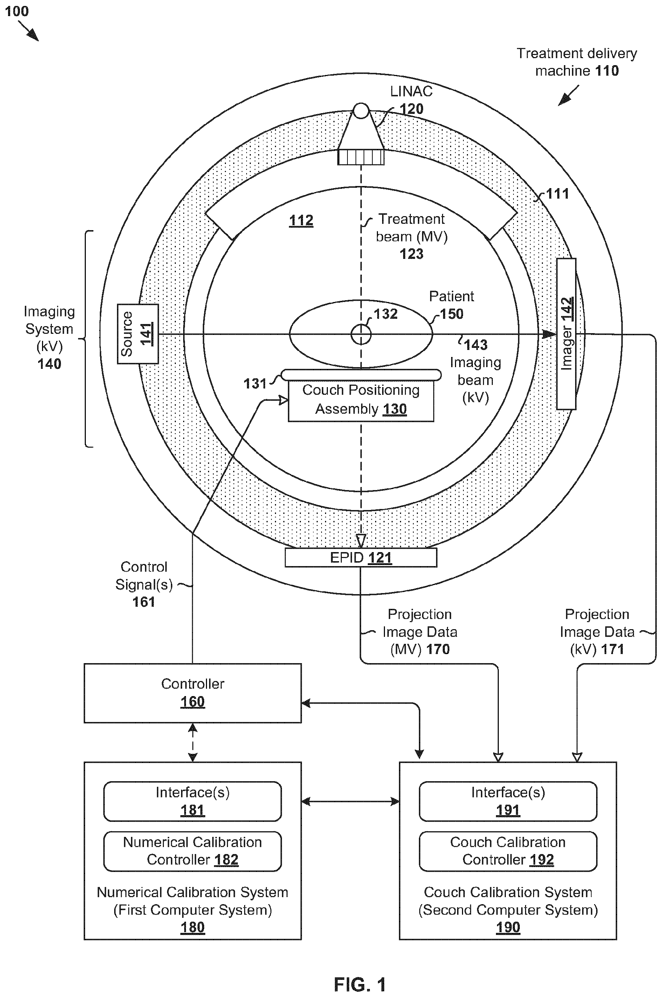

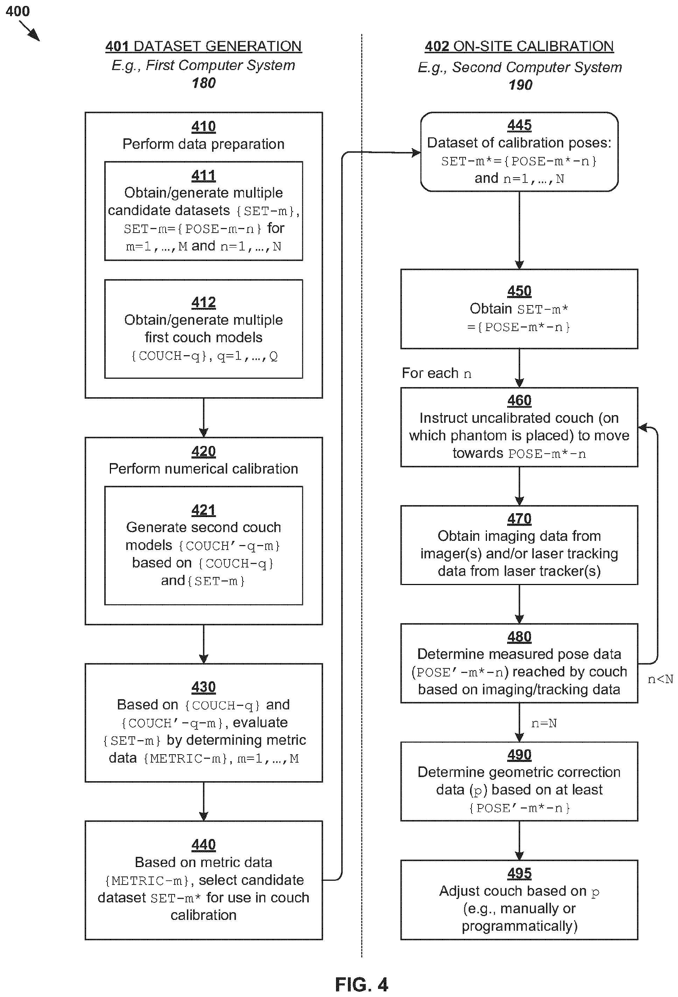

According to the first and second aspects, examples of the present disclosure provide method(s) and computer system(s) to perform a numerical calibration approach for treatment couch calibration. In one example, a computer system (e.g., numerical calibration system 180 in FIG. 1) may obtain multiple candidate datasets and multiple first couch models. For example, the multiple candidate datasets include at least (a) a first dataset of multiple first calibration poses and (b) a second dataset of multiple second calibration poses. Based on the multiple candidate datasets and the multiple first couch models, the computer system may perform numerical calibration to generate multiple second couch models. See also 410-420 in FIG. 4 (to be described below).

Based on the multiple first couch models and the multiple second couch models, the computer system may determine metric data associated with the multiple candidate datasets. The metric data may include at least (a) first metric data associated with the first dataset and (b) second metric data associated with the second dataset. The computer system may select, from the multiple candidate datasets, the first dataset based on the metric data. The first dataset may be selected for use by a controller or system (see 160/190 in FIG. 1) associated with a treatment couch (see 131 in FIG. 1) to cause the treatment couch to move towards each of the multiple first calibration poses during treatment couch calibration. The term "first dataset" may refer generally to any suitable dataset that is selected from the multiple candidate datasets and not necessarily to the initial dataset in the multiple candidate datasets. Note that any one of the multiple candidate datasets may be selected. See also 430-445 in FIG. 4 (to be described below). In another example, as an alternative to, or in addition to, the step of selecting from the multiple candidate datasets the first dataset based on the metric data, the method may comprise selecting, from the multiple candidate datasets, a dataset that is assessed to be most optimal. The optimality of each of the multiple candidate datasets may be assessed based on metric data such as calibration accuracy or calibration time when performing numerical calibration using the candidate dataset.

The methods and systems of the present disclosure may be used to perform treatment couch calibration. For example, the selected dataset such as the first dataset may be used to perform treatment couch calibration.

(b) Treatment couch calibration based on selected dataset

According to the third aspect, examples of the present disclosure provide a radiation therapy system (see 100 in FIG. 1) that includes at least (a) a treatment couch (see 131 in FIG. 1) that requires calibration, and (b) a couch calibration system (see 190 in FIG. 1). In one example, the couch calibration system may perform couch calibration by (a) obtaining a selected dataset of multiple calibration poses, and (b) performing treatment couch calibration based on the selected dataset according to any suitable approach. The dataset of multiple calibration poses may be selected (e.g., using numerical calibration system 180) from multiple candidate datasets based on metric data associated with the multiple candidate datasets.

For example, dataset selection may include (a) performing numerical calibration based on the multiple candidate datasets and multiple first couch models to generate multiple second couch models, and (b) generating the metric data based on the multiple first couch models and the multiple second couch models. See also 410-445 in FIG. 4 (to be described below). Treatment couch calibration based on the selected dataset may include the couch calibration system (a) instructing the treatment couch to move towards each of the multiple calibration poses in the selected dataset, (b) estimating measured pose data associated with the multiple calibration poses, and (c) determining geometric correction data associated with the treatment couch based on the measured pose data. See 450-490 in FIG. 4 (to be described below).

## DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented here. It will be readily understood that the aspects of the present disclosure, as generally described herein, and illustrated in the drawings, can be arranged, substituted, combined, and designed in a wide variety of different configurations, all of which are explicitly contemplated herein. Although the terms "first" and "second" are used to describe various elements, these elements should not be limited by these terms. These terms are used to distinguish one element from another. For example, a first element may be referred to as a second element, and vice versa. Independent of the grammatical term usage, individuals with male, female or other gender identities are included within the term.

Radiation therapy system

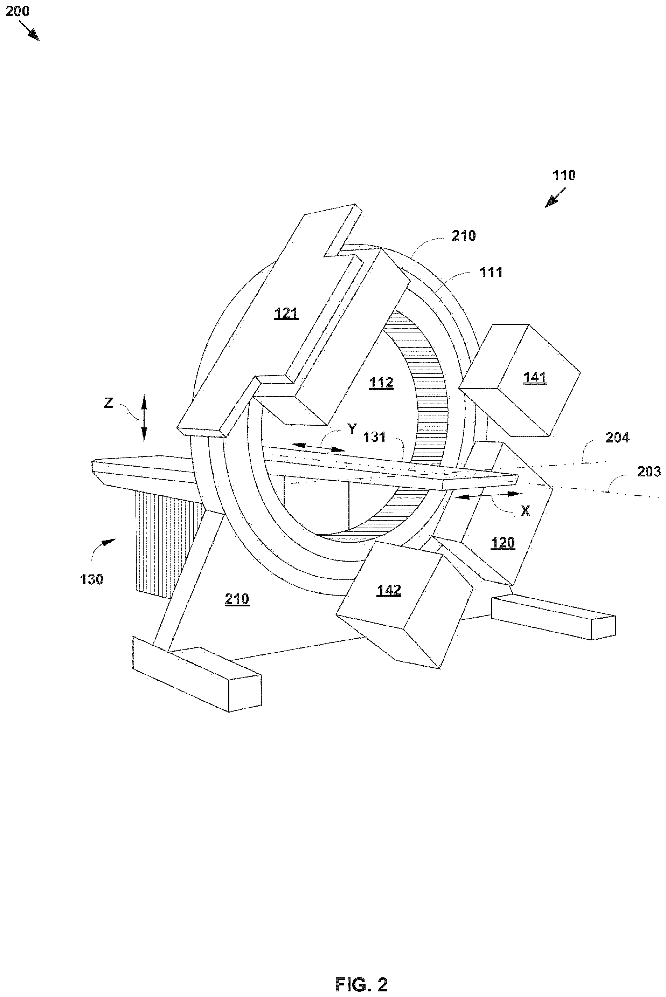

FIG. 1 is a schematic diagram illustrating example radiation therapy system 100 for delivery of radiation dose during a treatment phase of radiation therapy. FIG. 2 is a schematic diagram illustrating perspective view 200 of treatment delivery machine 110 in FIG. 1. It should be understood that, depending on the desired implementation, example system 100 and treatment delivery machine 110 may include additional and/or alternative components than that shown in FIGs. 1-2.

In the example in FIG. 1, radiation therapy system 100 may include (a) treatment delivery machine 110 to deliver treatment to patient 150, (b) control system or controller 160 to control operations of couch positioning assembly 130 to position patient 150, and (c) various computer systems 180-190 to facilitate treatment couch calibration according to examples of the present disclosure. Treatment delivery machine 110 may include gantry 111 that is rotatable about opening or bore 112 and treatment couch 131 for supporting patient 150 during radiation therapy. During treatment couch calibration, a calibration structure or device (known as a "phantom") may be positioned on treatment couch 131. As used herein, the term "treatment couch" may refer generally to a support structure (e.g., table) capable of supporting a patient or phantom. In practice, gantry 111 may have any suitable configuration, such as ring-based configuration (shown in FIGs. 1-2) or C-arm configuration (not shown).

Treatment delivery machine 110 may include a radiation source in the form of linear accelerator (LINAC) 120 as well as an imager/detector in the form of mega-electron volts (MV) electronic portal imaging device (EPID) 121. LINAC 120 may be configured to generate and direct treatment beam 123 towards isocenter 132 as gantry 111 is rotated through a treatment arc during VMAT. In practice, isocenter 132 may refer generally to a point at which beam trajectories associated with different gantry positions converge or intersect. Treatment beam 123 may be within a high-energy range, such as 1 MV or greater to deliver radiation doses to any suitable target structure(s) associated with patient 150, such as tumor, etc. Couch position assembly 130 may be configured to position treatment couch 131 and patient 150 during radiation therapy. Couch position assembly 130 may position treatment couch 131 when the system is offline, for example when radiation therapy is not being performed. For example, treatment couch 131 may be positioned such that a target structure (e.g., tumor) associated with patient 150 is at or near isocenter 132 about which LINAC 120, EPID 121, X-ray source 141, and X-ray imager 142 are rotated.

Treatment delivery machine 110 may further include imaging system 140 to facilitate kV imaging. Any suitable imaging modality or modalities may be used, such as cone beam computed tomography (CBCT), etc. Imaging system 140 may include at least one pair of kV imaging source 141 and kV imager 142. Compared to LINAC 120, kV imaging source 141 may be capable of producing imaging or diagnostic energy in the range of kV. To generate kV projection image data, kV imaging source 141 may be configured to emit and direct kV imaging beam 143 towards imager 142 to image patient 150. Although described with reference to MV LINAC 120 and MV treatment beam 123, it should be understood that any additional or alternative treatment delivery technique(s) may be used. For example, a proton treatment machine that includes a kV imaging system may be used instead.

Referring also to FIG. 2, various elements of treatment delivery machine 110 (e.g., LINAC 120, EPID 121, kV source 141, and kV imager 142) are shown to be partially rotated about treatment couch 131 at a specific point in time. Here, treatment delivery machine 110 may further include a drive stand 210 that is mechanically coupled to couch positioning assembly 130. In practice, drive stand 210 may be a fixed support structure for components of treatment delivery machine 110, including gantry 111, a drive system (not shown) for rotatably moving gantry 111, cooling systems (not shown) of treatment delivery machine 110, etc. Drive stand 210 may rest on and/or be fixed to a support surface, such as a floor of a treatment facility. In the example in FIG. 2, couch positioning assembly 130 may be mechanically coupled to drive stand 210 and configured to adjustably position treatment couch 131 relative to bore 112 and LINAC 120. Treatment couch 131 is shown to be longitudinally extending from couch positioning assembly 130 into a bore 112 of treatment delivery machine 110.

Couch positioning assembly

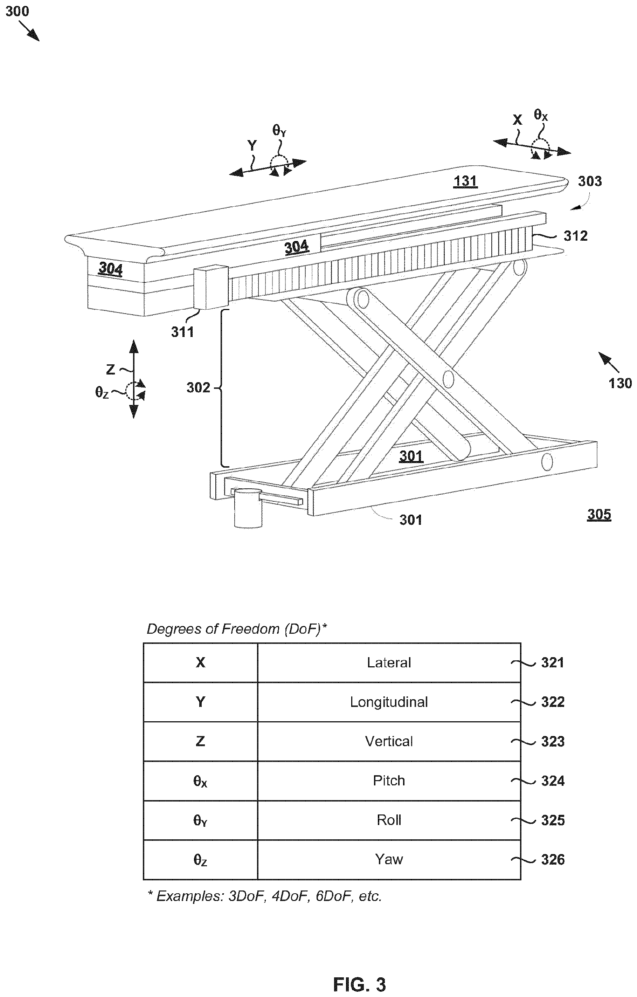

FIG. 3 is a schematic diagram illustrating example detailed view 300 of couch positioning assembly 130 in the example in FIGs. 1-2. Here, couch positioning assembly 130 is depicted without external panels (shown in FIG. 2) for clarity. In the example in FIG. 3, couch positioning assembly 130 may include lift base 301 on which scissor lift mechanism 302 and longitudinal frame 303 are mounted. In this example, lift base 301 may be configured to translate treatment couch 131 in lateral directions (X). Coupled to longitudinal frame 303 is a longitudinal carriage 304 that may be translated along longitudinal directions (Y) via longitudinal motor 311. Treatment couch 131 may be mounted on, and longitudinally moved by, longitudinal carriage 304. Scissor lift mechanism 302 may be configured to translate treatment couch 131 in vertical directions (Z) using any technically feasible actuation system.

Depending on the desired implementation, couch positioning assembly 130 and treatment couch 131 may support movements in any suitable number of degrees of freedom (DoF), such as three (3DoF), four (4DoF), six (6DoF), etc. In the case of 3DoF, couch positioning assembly 130 may be configured to translate treatment couch 131 along (X, Y, Z) directions, where X = lateral directions (see 321), Y = longitudinal directions (see 322), and Z = vertical directions (see 323). The three primary axes are also shown in FIG. 2. Movement of treatment couch 131 along lateral directions X corresponds to horizontal movement towards one side or the other side (e.g., left or right) of bore 112. Movement along longitudinal directions Y corresponds to horizontal movement into or out (e.g., forward or backward) of bore 112. Movement along vertical directions Z corresponds to vertical movement towards or away from (e.g., down or up) treatment room floor 305.

In the case of 4DoF, movements about one rotational axis are supported in addition to three translational axes. In this case, couch positioning assembly 130 may be configured to position treatment couch 131 along (X, Y, Z, θ) directions, where θ = pitch associated with rotation about a lateral (X) axis, θ = rotation (e.g. roll) about the Y-axis, or θ = yaw associated with rotation about the Z-axis. Compared to 3DoF, a 4DoF couch provides more flexibility in adjusting patient position, especially when dealing with rotational errors. See corresponding 321-323 and 324/325/326 in FIG. 3.

In the case of 6DoF, movements about three translational axes and three rotational axes are supported. In this case, couch positioning assembly 130 may be configured to position treatment couch 131 along $(X, Y, Z, \theta_X, \theta_Y, \theta_Z)$ directions, where $\theta_X$ = pitch associated with rotation about the lateral X-axis, $\theta_Y$ = roll associated with rotation about the longitudinal Y-axis, and $\theta_Z$ = yaw associated with rotation about the vertical Z-axis. Compared to a 3DoF or 4DoF couch, a 6DoF couch may combine pitch, roll and yaw rotational motions with translational motions to achieve high-precision patient positioning, such as in the order of submillimeter and sub-degree precision. See corresponding 321-326 in FIG. 3.

In practice, couch positioning assembly 130 may include any suitable motors and joints (not all shown in FIG. 3 for simplicity). To support movement in lateral directions X, couch positioning assembly 130 may include a lateral movement motor (e.g., linear motor) for selectively moving treatment couch 131 in lateral directions X. To support movement in longitudinal directions Y, couch positioning assembly 130 may include a longitudinal movement motor (e.g., linear motor) that is coupled with longitudinal frame 303 for selectively moving treatment couch 131 in longitudinal directions Y. To support movement in vertical directions Z, couch positioning assembly 130 may include scissor lift mechanism 302 for moving treatment couch 131 using any suitable actuation system, such as may be an electric motor, a hydraulic actuator, a stepper motor, etc. To support rotational motions in $(\theta_X, \theta_Y, \theta_Z)$ directions, couch positioning assembly 130 may include any suitable rotational motors to enable pitch, roll and yaw adjustments.

Couch positioning assembly 130 may be controlled using controller 160 in FIG. 1 using any suitable commands and control signals (see 161 in FIG. 1). For quality assurance (QA) purposes or in general, treatment couch calibration may be performed prior to treatment delivery, for example such that the actual movement of treatment couch 131 better matches with or follows the commands from controller 160. For example, when 6DoF couch 131 is instructed to move towards a particular target position (known as "pose"), the reached position might differ from the target position. In an example, a "pose" may correspond to particular values of the lateral, longitudinal and/or vertical positions of the couch 131, and particular values of the pitch, roll and/or yaw of the couch 131. The discrepancy may be due to various imperfections associated with couch 131. This is undesirable because, in some cases, even minor discrepancies may lead to inaccurate radiation delivery to a target structure (e.g., tumor) and inadvertently harm healthy tissues of patient 150.

In practice, one challenge of treatment couch calibration is the high dimensionality of the process, particularly because a large number of manual calibration measurements are usually required. For example, when attempting to calibrate treatment couch 131 having 6DoF, one natural approach is to use a measurement grid to sample each of the six translational and rotational axes in every combination. This grid-based sampling approach leads to a large number of calibration measurements, even when coarse sampling is used. For example, a 6-dimensional measurement grid with three (3) steps for each axis would necessitate $3^6$ = 729 measurements. Assuming an approximate time of about 15 seconds per pose, the calibration time would be more than three hours for 729 calibration poses. As the sampling granularity increases, more calibration time is required. For example, a measurement grid with five (5) instead of three steps per axis would necessitate $5^6$ = 15625 measurements. In this case, the calibration time would increase to around 65 hours. In practice, substantially long calibration time (e.g., 3 or 65 hours in the examples provided) is inefficient and undesirable.

Numerical calibration approach

According to examples of the present disclosure, treatment couch calibration may be implemented more efficiently. Instead of relying on conventional grid-based sampling, a numerical calibration approach may be implemented to select a dataset of calibration poses for treatment couch calibration. Examples of the present disclosure may be implemented to alleviate challenge(s) relating to couch calibration. A first challenge is to determine the size of the dataset (i.e., how many calibration poses are required). A second challenge is to select particular calibration poses to form the dataset (i.e., which calibration poses). In practice, the dataset may be selected based on any desired selection criterion or criteria relating to calibration accuracy and/or calibration time.

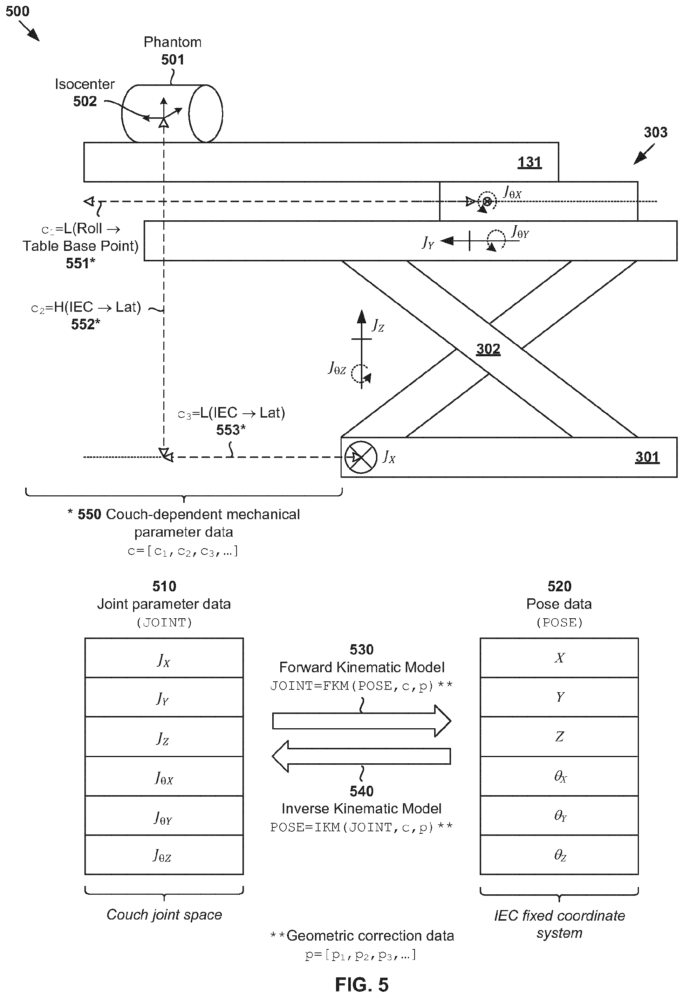

As used herein, the term "pose" may refer generally to a position associated with a treatment couch. The term "calibration pose" or "calibration measurement position" may refer generally to a position that a treatment couch is instructed to reach during couch calibration. The term "reached pose" or "measured pose" may refer generally to a position that is actually achieved by the treatment couch, as measured using any suitable approach (e.g., imaging data 170/171 from imager 121/142, tracking data from laser tracker(s), etc.). As discussed above, there may be discrepancy between a calibration pose (i.e., target pose) and a measured pose due to various couch imperfections. As will be explained using FIG. 5, a calibration pose may be expressed using coordinates in a machine coordinate space, such as the International Electrotechnical Commission (IEC) fixed coordinate system, etc.

Some examples will be described using FIG. 4, which is a flowchart of example process 400 for first computer system 180 to perform a numerical calibration approach for calibration dataset generation and second computer system 190 to perform treatment couch calibration. Example process 400 may include one or more operations, functions, or actions illustrated by one or more blocks, such as 410 to 495. Depending on the desired implementation, various blocks may be combined into fewer blocks, divided into additional blocks, and/or eliminated. In the example in FIG. 4, treatment couch calibration may include two phases: (a) calibration dataset generation 401 to generate/select a dataset of calibration poses (see blocks 440-445) and (b) on-site calibration 402 based on the generated dataset (see blocks 450-495).

Examples of the present disclosure may be implemented using any suitable computer system(s). As used herein, the term "computer system" may refer generally to one or more physical machines (bare metal machines), or virtual machines (VMs) deployed in a cloud-based environment. Referring to FIG. 1 again, computer systems 180-190 may be communicatively coupled with treatment delivery machine 110 to facilitate couch calibration. Note that first and second computer systems 180-190 may be implemented using one or more physical/virtual machines. Further, computer system 180/190 may be located in the same, or a different, physical location as treatment delivery machine 110. In a further example, the first and second computer systems 180, 190 may be part of the same overall computer system.

Using the example in FIG. 1, first computer system 180 (i.e., numerical calibration system) may be configured to perform calibration dataset generation 401 according to blocks 440-445 in FIG. 4. First computer system 180 may include any suitable hardware and/or software components, such as interface 181 to interact with controller 160 and/or second computer system 190, and numerical calibration controller 182 to perform blocks 410-440. Second computer system 190 (i.e., couch calibration system) may be configured to perform treatment couch calibration 402 according to blocks 450-495 in FIG. 4. Second computer system 190 may include any suitable hardware and/or software components, such as interface 191 to interact with controller 160 and/or first computer system 180, couch calibration controller 192 to perform blocks 450-495. Phases 401-402 will be described in turn below.

(a) Dataset generation (see 401)

At 410 in FIG. 4, first computer system 180 may perform data preparation by obtaining or generating multiple ($M$) candidate datasets and multiple ($Q$) first couch models. The term "obtaining" or "obtain" may refer generally to retrieving data from another computer system or datastore. Each candidate dataset may be denoted as $SET\text{-}m$ to represent a set of multiple ($N$) calibration poses ($POSE\text{-}m\text{-}n$), where $SET\text{-}m$ = {$POSE\text{-}m\text{-}n$} for $m$ = 1, ... , $M$ and $n$ = 1, ... , $N$. For example, {$SET\text{-}m$} may include a first dataset ($SET\text{-}1$) that includes multiple first calibration poses, and a second dataset ($SET\text{-}2$) that includes multiple second calibration poses. See 411 in FIG. 4.

The multiple first couch models may be denoted as {$COUCH\text{-}q$} for $q$ = 1, ... , $Q$. Each first couch model ($COUCH\text{-}q$) may represent a simulated couch, which may be used as a ground truth couch model during subsequent numerical calibration at block 420. As used herein, the term "couch model" may refer generally to a representation or description of a physical treatment couch using any suitable data. Poses reached by a particular *COUCH-q* may include any suitable random offset to simulate measurement inaccuracy during the measurement process. According to examples of the present disclosure, a couch model may be represented using geometric correction data to represent deviation(s) of a couch from an ideal couch model. See 412 in FIG. 4.

At 420 in FIG. 4, based on {*SET-m*} and {*COUCH-q*}, first computer system 180 may perform numerical calibration to generate multiple ($Q \times M$) second couch models denoted as {*COUCH'-q-m*}. Each second couch model (*COUCH'-q-m*) may represent a couch model that is obtained through numerical calibration based on a particular first couch model (*COUCH-q*) and a particular candidate dataset (*SET-m*). Numerical calibration may be performed to generate a calibrated couch model (*COUCH'-q-m*) that is as close as possible to its corresponding ground truth model (*COUCH-q*), such as using an optimization process. Depending on the desired implementation, block 420 may involve estimating measured or reached pose based on a kinematic model associated with a particular first couch model (to be explained using FIG. 5). See also 421-422 in FIG. 4.

At 430 in FIG. 4, based on the multiple first couch models (i.e., {*COUCH-q*}) and the multiple second couch models (i.e., {*COUCH'-q-m*})*,* first computer system 180 may evaluate the multiple candidate datasets by determining metric data associated with the multiple candidate datasets. For example, the metric data (denoted as *METRIC-m*) may include first metric data (*METRIC-1*) and second metric data (*METRIC-2*) associated with the respective first dataset (*SET-1*) and second dataset (*SET-2*). Here, the term "metric data" may refer generally to a quantitative measure to assess or quantify the quality of each candidate dataset. Example metric data may include statistical measures, such as mean deviation data, maximum deviation data, deviation range data, etc.

At 440 in FIG. 4, based on at least the metric data, first computer system 180 may select a particular dataset (denoted as *SET-m\**) from {*SET-m*}*.* For example, the selected dataset may be the first dataset (e.g., *SET-*1, $m^* = 1$) for use by controller 160 to cause the treatment couch to move towards each of the first calibration poses during couch calibration. The term "first dataset" may refer generally to any suitable dataset (*SET-m*) that is selected from {*SET-m*} based on {*METRIC-m*}*.* Note that the first dataset is not necessarily *SET-*1*.* See also 445 in FIG. 4.

As will be explained further below, examples of the present disclosure may be implemented using numerical simulations of anticipated imperfections associated with treatment couches to be calibrated to select a desired (e.g., optimal) dataset of calibration poses. The multiple candidate datasets may be tested using the multiple first couch models that represent different couch imperfections. The multiple candidate datasets may then be assessed based on any suitable metric data, such as how accurate the second couch models are after numerical calibration. This way, one candidate dataset may be selected for use during subsequent couch calibration 402.

(b) Couch calibration based on selected dataset (see 402)

Selected dataset (*SET-m\**) 445 may be used to calibrate particular treatment couch 131 using any suitable approach. In one example, at 450-460 in FIG. 4, based on dataset 445 obtained from first computer system 180 or any suitable datastore, second computer system 190 may instruct controller 160 to cause uncalibrated treatment couch 131 to move towards each calibration pose in dataset 445, i.e., *POSE-m\*-n* $\in$ *SET-m\** for $n = 1, ... , N$. At 470-480, for each *POSE-m\*-n,* second computer system 190 may determine measured pose data (denoted as *POSE'-m\*-n*) reached by treatment couch 131, such as based on imaging data (e.g., MV and/or kV projection image data 170/171) and/or laser tracking data, etc.

At 490 in FIG. 4, second computer system 190 may determine geometric correction data based on the measured pose data ({*POSE'-m\*-n*}). This way, at 495, treatment couch 131 may be adjusted using any suitable approach. In one example, manual adjustments may be performed on treatment couch 131 by a technician. In another example, second computer system 190 may adjust treatment couch 131 programmatically, such as using a kinematic calibration approach. Some examples of the kinematic calibration approach are described in related European Patent Application entitled "Kinematic Calibration of a Treatment Couch for Radiation Therapy", (Attorney Case No. 09679) with priority date 20 August 2024, and priority application number 18/809,573, and not repeated here for brevity.

Kinematic Modelling

According to examples of the present disclosure, treatment couch calibration may be performed based on a kinematic model. In general, the term "kinematics" may refer generally to a branch of robotics or mathematics that focuses on describing the motion of objects. As used herein, the term "kinematic model" may refer generally to a representation (e.g., robotics or mathematical representation) that describes the motion of a moveable system, such as robot or treatment couch 131 for radiation therapy. In the following, various examples will be explained using treatment couch 131 supporting 6DoF (see 321-326 in FIG. 3). In practice, examples of the present disclosure may be implemented for treatment couch 131 supporting any degrees of freedom, such as 3DoF, 4DoF, etc.

An example will be explained using FIG. 5, which is a schematic diagram illustrating example kinematic modeling 500 for treatment couch calibration. Here, a side view of treatment couch 131 and couch positioning assembly 130 is shown. One goal of treatment couch calibration may include determining a kinematic model that describes, as close as possible to reality, a relationship or conversion between (a) couch motor and/or joint settings associated with treatment couch 131 and (b) its so-called end-effector pose. In an example, conversion from couch motor and/or joint settings to an end-effector pose may be referred to as a forward kinematic model. In an example, conversion from an end-effector pose to couch motor and/or joint settings may be referred to as an inverse kinematic model. That means, for a desired end-effector pose, each couch joint may move in such a way that the pose is reached within certain tolerance values.

(a) Couch joint space

At 510 in FIG. 5, couch motor and/or joint parameter data (denoted as *JOINT*) may be expressed in a couch joint space. For brevity, the term "couch joint parameter data" will be used to represent couch motor and/or joint settings. One example is $JOINT = (J_X, J_Y, J_Z, J_{\theta X}, J_{\theta Y}, J_{\theta Z})$, where $(J_X, J_Y, J_Z)$ may represent motor or joint parameter data associated with translational motion in the lateral direction *X,* longitudinal direction *Y* and vertical direction *Z* in the couch joint space, respectively. Further, $(J_{\theta X}, J_{\theta Y}, J_{\theta Z})$ may represent motor or joint parameter data associated with rotational motion about a lateral axis, a longitudinal axis and a vertical axis in the couch joint space, respectively. As described using FIG. 3, couch positioning assembly 130 may include lift base 301, scissor lift mechanism 302 and longitudinal frame 303 to move treatment couch 131 in translational and rotational directions.

(b) IEC fixed coordinate system

At 520 in FIG. 5, an end-effector pose (denoted as *POSE*) may be expressed in the IEC fixed coordinate system. One example is $POSE = (X, Y, Z, \theta_X, \theta_Y, \theta_Z)$, where $(X, Y, Z)$ may represent translational movements in respective lateral axis *X,* longitudinal direction *Y* and vertical direction Z in the IEC fixed coordinate system. Further, $(\theta_X, \theta_Y, \theta_Z)$ may be angles representing rotational movements about respective lateral *X*-axis, longitudinal *Y*-axis and vertical *Z*-axis in the IEC fixed coordinate system, respectively. In practice, one way to model the end-effector pose is by way of the origin and orientation of a calibration phantom (see 501 in FIG. 5) that is placed on treatment couch 131.

(c) Forward and inverse kinematic models

A kinematic model describes the conversion between (a) couch joint parameter data (*JOINT*) in the couch joint space and (b) an end-effector pose (*POSE*) in the IEC fixed coordinate system in both directions. At 530 in FIG. 5, a forward kinematic model (FKM) for mapping or converting *JOINT* in the couch joint space to *POSE* in the IEC fixed coordinate system may be expressed as follows using c = mechanical parameter data and *p* = geometric correction data (to be described below) associated with treatment couch 131:

$$\text{FKM}(JOINT, c, p) = \text{FKM}(J_X, J_Y, J_Z, J_{\theta X}, J_{\theta Y}, J_{\theta Z}, c, p) = (X, Y, Z, \theta_X, \theta_Y, \theta_Z) = POSE.$$

At 540 in FIG. 5, an inverse kinematic model (IKM) for mapping or converting (a) an end-effector pose (*POSE*) in the IEC fixed coordinate system to (b) couch joint parameter data (*JOINT*) in the couch joint space may be expressed as follows using *c* = mechanical parameter data and *p* = geometric correction data (to be described below) associated with treatment couch 131:

$$\text{IKM}(POSE, C, p) = \text{IKM}(X, Y, Z, \theta_X, \theta_Y, \theta_Z, c, p) = (J_X, J_Y, J_Z, J_{\theta X}, J_{\theta Y}, J_{\theta Z}) = JOINT.$$

(e) Mechanical parameter data

At 550 in FIG. 5, mechanical parameter data (*c*) for the FKM and IKM may include any parameter(s) associated with treatment couch 131, such as nominal geometric relations between various joints. At 551, a first example (*c*1) is H(*IEC→Lat*), which represents the height (H) or vertical distance from an isocenter in the IEC fixed coordinate system (see 502 in FIG. 5) to a lateral joint. At 552, a second example (*c*2) is L(*IEC→Lat*), which represents a longitudinal distance from the isocenter (*IEC*) to a lateral joint (*Lat*). At 553, a third example (c3) is L(*Roll→Base Point*), which represents a longitudinal distance between a roll joint (*Roll*) and a tabletop base point (*Base Point*) of treatment couch 131. Other examples may include H(*Lng→Pitch*) = vertical distance from a longitudinal joint (*Lng*) to a pitch joint (*Pitch*), L(*Vrt→Lng*) = longitudinal distance from a vertical joint (*Vrt*) to a longitudinal joint (*Lng*), etc.

In practice, the mechanical parameter data is different for each individual couch. While the mechanical parameter data is expected to stay within defined tolerances of the manufacturing process of treatment couch 131, some deviations from nominal values are expected. These deviations may be caused by various imperfections, such as offsets from the nominal lengths of parts of treatment couch 131, misaligned axes (e.g., longitudinal motion direction is not orthogonal to the vertical motion direction), a misaligned object on treatment couch 131 (i.e., the end-effector). To account for these deviations, geometric corrections may be introduced to the kinematic model. These geometric corrections, which are specific for individual couches, are unknown parameters in the model. The process of couch calibration may include finding these geometric corrections.

(f) Geometric correction data

As used herein, the term "geometric correction data" or "geometric parameter data" associated with a couch may refer generally to one or more linear and/or rotational adjustment(s) that may be made to the couch. In practice, a particular geometric correction (denoted as $p$) may be associated with a position of a component of couch positioning assembly 130, a distance between two components (e.g., joint-to-joint distance), an orientation of a component, an axis of rotation, a center of rotation, a distance between an isocenter to a particular component, etc. A first example geometric correction ($p_1$) may be an offset to a linear or angular distance (e.g., 0.5 mm or 0.2 degrees) between isocenter 502 and a lateral joint of treatment couch 131. A second example ($p_2$) may be an offset to a distance (e.g., 0.5 mm) between a yaw joint and a vertical joint. A third example ($p_3$) may be an offset to a distance (e.g., 0.5 mm) between a pitch joint and a roll joint. A fourth example ($p_4$) may be an offset to a linear or angular distance (e.g., 0.5 mm or 0.2 degree) between a roll joint and a table top base point. A fifth example ($p_5$) may be an offset to an angular distance (e.g., 0.2 degree) between a vertical joint and a longitudinal joint.

In practice, the forward and inverse kinematic models may include any suitable transformation functions or matrices for transform *JOINT* into *POSE,* and vice versa. For example, given a moveable system (e.g., robot or couch) with three so-called prismatic joints (e.g. moving in lateral, longitudinal and vertical directions), the model may be described as follows. Let $d_k$ = movable joint variable of a particular joint $k$ and $p_k$ = geometric correction(s), the forward kinematic can be described as: $f(d_1, d_2, d_3, p) = A_1(d_1, p_1) * A_2(d_2, p_2) * A_3(d_3, p_3)$, where $A_i$ is the transformation matrix of joint $k$.

For instance, using the Denavit-Hartenberg representation in the field of robotics, each joint may be described by one free parameter (e.g., $d_k$) and two fixed parameters, such as link length offset $a_k$ (i.e. the distance between two consecutive joints in the chain), and link twist offset $\alpha_k$ (i.e. the angle between the common normal of two consecutive joints). The parameters $p_k$ may be, for instance, offsets to the fixed parameters $a_k$ and $\alpha_k$. For the inverse kinematics and a requested end-effector pose $(X_r, Y_r, Z_r)$, a set of equations may be defined based on the forward kinematics as $f(d_1, d_2, d_3, p) = (X_r, Y_r, Z_r)$. This equation system may be solved numerically, as a closed-form analytical solution is generally not available when geometric corrections are involved. These examples discussed with reference to the lateral, longitudinal and vertical directions are also applicable to rotational directions.

Detailed example

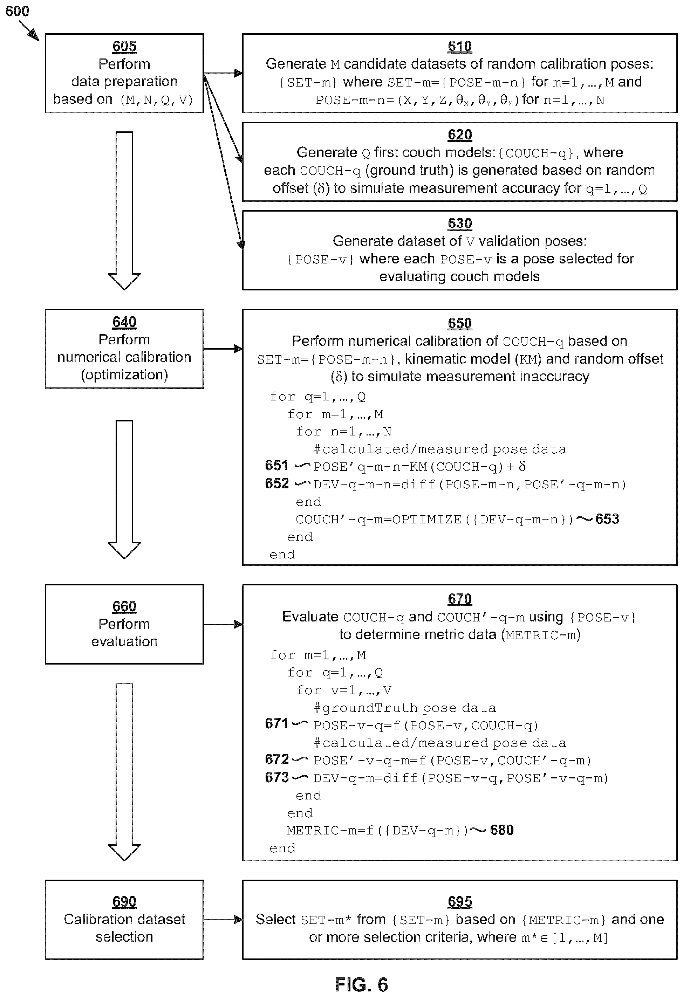

FIG. 6 is a flowchart of example detailed process 600 for first computer system 180 to perform a numerical calibration approach for treatment couch calibration. Example process 600 may include one or more operations, functions, or actions illustrated by one or more blocks, such as 605 to 695. Depending on the desired implementation, various blocks may be combined into fewer blocks, divided into additional blocks, and/or eliminated.

(a) Data preparation

At 605 in FIG. 6, computer system 180 may perform data preparation based on parameters = $(M, N, Q, V)$ that are configurable by a user (e.g., technician, clinician). Here, $M$ = number of candidate datasets to be generated, $N$ = number of calibration poses within each candidate dataset, $Q$ = number of first couch models to be simulated and V = number of validation poses to be generated for evaluation purposes. Data preparation may include blocks 610-630 below.

At 610 in FIG. 6, computer system 180 may generate or obtain multiple (M) candidate datasets denoted as *{SET-m}*, where *SET-m* = *{POSE-m-n}* for m = 1, ... , M and n = 1, ... , N. Here, each candidate dataset *(SET-m)* may include multiple (N) calibration poses, each being denoted as *POSE-m-n = (X, Y, Z, $\theta_X$, $\theta_Y$, $\theta_Z$)* in the IEC coordinate space. Any suitable approach may be used to generate *SET-m,* such as random generation of calibration poses *{POSE-m-n}*, etc. In practice, the calibration poses may be generated randomly within a defined volume relevant to a particular application, such as by considering collision avoidance, visibility of the measurement object (e.g., phantom), etc.

At 620 in FIG. 6, computer system 180 may generate or obtain multiple (Q) first couch models denoted as *{COUCH-q}*, where q = 1, ... , Q, such as based on a ground truth kinematic model. As explained using FIG. 4, poses reached by *COUCH-q* may include any suitable random offset ($\delta$) to simulate measurement inaccuracy during the measurement process. Each first couch model *(COUCH-q)* may be associated with a geometric correction set that is applicable to an ideal couch model. Each geometric correction set may be simulated based on expected geometric errors of real couches. Each first couch model (*COUCH-q*) represents a ground truth model for treatment couch 131.

At 630 in FIG. 6, computer system 180 may generate or obtain multiple (V) validation poses denoted as {*POSE-v*}, where $v = 1, \ldots, V$. Each validation pose (*POSE-v*) may be selected such that the required accuracy specification(s) associated with treatment couch 131 is verifiable. Compared to {*POSE-m-n*} that may be randomly generated, {*POSE-v*} may represent a fixed number (*V*) of validation poses that spread over a full or partial travel range associated with treatment couch 131.

(b) Numerical calibration

At 640 in FIG. 6, computer system 180 may perform numerical calibration based on multiple (*M*) candidate datasets and multiple (Q) first couch models according to blocks 650-653. For example, at 650, for each $q \in [1, \ldots, Q]$ and $m \in [1, \ldots, M]$, computer system 180 may perform numerical calibration of a particular first couch model *(COUCH-q)* based on a particular candidate dataset (*SET-m*). This will be repeated for all $q = 1, \ldots, Q$ and $m = 1, \ldots, M$. This way, each calibration dataset *SET-m* may be evaluated based on all *Q* simulated first couch models.

At 651, for each calibration pose (*POSE-m-n*) in the particular candidate dataset (*SET-m*), measured pose data (denoted as *POSE'-q-m-n*) may be simulated. Here, *POSE'-q-m-n* may be determined based on a kinematic model (*KM*) associated with the first couch model *(COUCH-q)* and adding a random accuracy offset ($\delta$), i.e., *POSE'-q-m-n* = *KM(COUCH-q)* + $\delta$. The random offset, which is part of the measurement process, may be configured to be within an expected measurement accuracy to account for measurement uncertainty. At 652, deviation data associated with (*POSE-n, POSE'-q-m-n*) for a particular *COUCH-q* and *SET-m* may be determined as follows:

$$DEV\text{-}q\text{-}m\text{-}n = diff(POSE\text{-}m\text{-}n, POSE'\text{-}q\text{-}m\text{-}n), \text{ where } POSE\text{-}m\text{-}n \in SET\text{-}m.$$

At 653, second couch model *(COUCH'-q-m)* may be generated based on deviation data associated with (*POSE-m-n, POSE'-q-m-n*) for $n = 1, \ldots, N$. For example, *COUCH'-q-m* may be generated by solving an optimization problem to determine geometric correction data associated with *COUCH'-q-m* that minimizes {*DEV-q-m-n*} for $n = 1, \ldots, N$. The optimization process is represented using an OPTIMIZE function for finding the optimal second couch model (*COUCH'-q-m*) such that the deviation between modelled and measured poses is minimal.

Depending on the desired implementation, optimization may be implemented at block 650 using any suitable approach, such as numerical simulation using Monte Carlo simulation, simulated annealing approach, conjugate gradient approach, linear programming, dynamic programming, machine learning approach, etc. Various optimization approaches may be found in Numerical Recipes: The Art of Scientific Computing (by W. H. Press, S. H. Teukolsky, W. T. Vetterling and B. P. Flannery), which is incorporated herein by reference.

(c) Evaluation

At 660 in FIG. 6, computer system 180 may evaluate each second couch model (*COUCH'-q-m*) by applying it to each validation pose *(POSE-v)* according to block 670. At 671, for each $m \in [1, \ldots, M]$, $q \in [1, \ldots, Q]$ and $v \in [1, \ldots, V]$, computer system 180 may determine ground truth pose data denoted as *POSE-v-q* = *f(POSE-v, COUCH-q)*. Here, *POSE-v-q* may represent a particular ground truth pose that is determined as a function of a validation pose (*POSE-v*), a particular first couch model (*COUCH-q*), and any suitable random offset to account for measurement uncertainty (not shown in FIG. 6 for simplicity).

Next, at 672 in FIG. 6, computer system 180 may determine calculated/measured pose data denoted as *POSE'-v-q-m* = *f(POSE-v, COUCH'-q-m)*. Here, *POSE'-v-q-m* may represent a measured pose that is determined as a function of a particular validation pose *(POSE-v)* and a particular second couch model *(COUCH'-q-m)* from blocks 640-650.

At 673 in FIG. 6, error or deviation data (*DEV-q-m*) associated with a particular *COUCH-q* and *SET-m* may be generated as follows by calculating a difference between (a) the ground truth pose data (*POSE-v-q*) and (b) the measured pose (*POSE'-v-q-m*) data as follows:

$$DEV\text{-}q\text{-}m = diff(POSE\text{-}v\text{-}q, POSE'\text{-}v\text{-}q\text{-}m).$$

At 680 in FIG. 6, for each $m \in [1, \ldots, M]$, computer system 180 may determine metric data (denoted as *METRIC-m*) associated with each candidate dataset *SET-m* based on a set of deviation data = {*DEV-q-m*} for $q = 1, \ldots, Q$. The metric data may be calculated to include any suitable statistical measure(s), such as mean or average deviation data, maximum deviation data, deviation range data, etc.

(d) Dataset selection

At 690-695 in FIG. 6, computer system 180 may perform calibration dataset selection by comparing {*SET-m*} based on their {*METRIC-m*}, and selecting a particular candidate dataset (*SET-m\**), where m* $\in$ [1, ..., *M*]. The selection may be performed based on any suitable selection criterion or criteria, such as calibration accuracy that depends on the quality of calibration poses in the selected dataset (*SET-m\**), calibration time that depends on the quantity (i.e., size *N*) of calibration poses {*POSE-m-n*} in *SET-m\*,* or any combination thereof.

Example numerical calibration

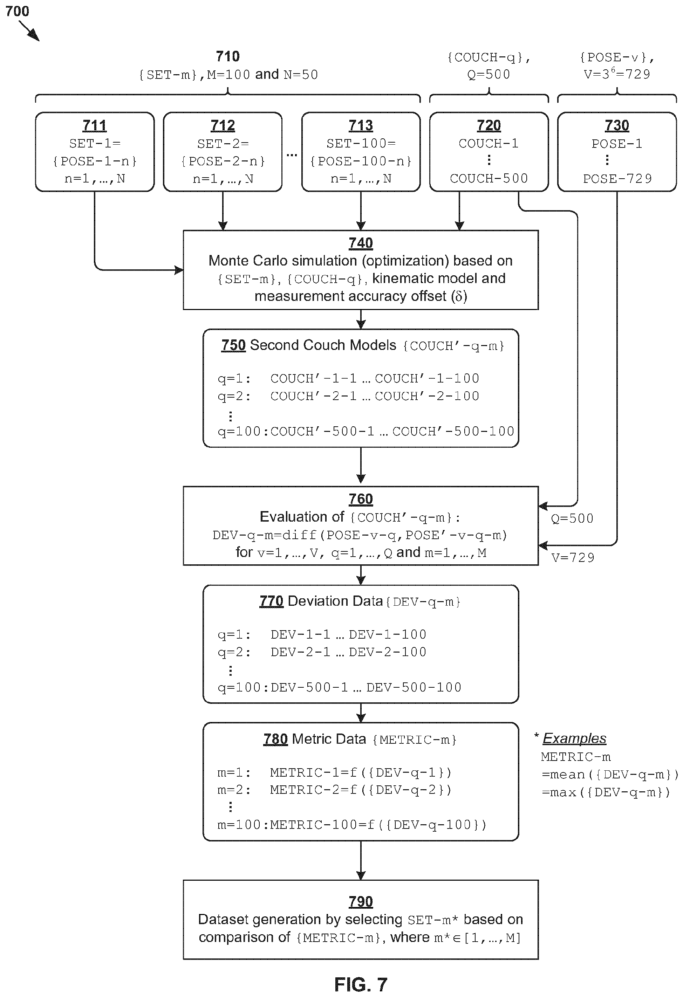

In practice, to determine a realistic estimate metric data (*METRIC-m*) indicating the calibration performance associated with a particular candidate dataset (*SET-m*), the dataset may be tested on many (*Q*) different simulated couch models and *V* validation poses. For example, the "best" or "optimal" dataset may be selected based on calibration accuracy, as evaluated by numerically calibrating *Q* first couch models based on *V* validation poses. An example will be explained using FIG. 7, which is a schematic diagram illustrating example numerical calibration approach 700 for calibration dataset generation according to the example in FIG. 6. In this example, the following parameters may be configured: *M* = 100, *N* = 50, *Q* = 500, *V* = 729. Note that blocks 710-790 in FIG. 7 correspond with respective blocks 610-695 in FIG. 6. Any suitable *M*, *N*, *Q* and *V* may be used in practice.

(a) Monte Carlo simulations

At 710 in FIG. 7, *N* = 50 calibration poses in each candidate dataset *(SET-m)* may be selected randomly within predefined ranges associated with ($X$, $Y$, $Z$, $\theta_X$, $\theta_Y$, $\theta_Z$). Example translational ranges may include $\pm$ 20 cm in the lateral direction ($X$), $\pm$ 20 cm in the longitudinal direction ($X$) and $\pm$ 20 cm in the vertical direction ($Z$). Example rotational ranges may include $\pm$ 3 degrees around all three axes through isocenter 502 at a couch position such that a center of phantom 501 is aligned with isocenter 502 (e.g., points that may either lead to insufficient markers in the image or collisions were excluded). See *SET*-1 to *SET*-100 at 711-713 in FIG. 7.

At 720 in FIG. 7, *Q* = 500 first couch models may be simulated. Each first couch model (*COUCH-q*) may be associated with a random geometric correction set to model linear and/or rotational deviations from an ideal couch. In practice, the geometric corrections are specific for individual couches. The geometric corrections may be simulated as random values within an expected range, such as $\pm$ 0.5 mm for linear quantities and $\pm$ 0.1 degree for rotational quantities. These ranges may be designed based on expected deviations, such as expected deviations in the manufacturing process.

At 730 in FIG. 7, $V = 3^6 = 729$ validation poses may be simulated to evaluate the accuracy of a couch model. In practice, the validation poses should be different to the calibration poses used for numerical calibration. Any suitable approach may be used to generate the validation poses. Using a grid-based approach, for example, all combinations of $\pm$ 5 cm shifts along each translational axis and $\pm$ 3 degrees along each rotational axis (i.e., three points per each of six axes) may be generated. *V* = 729 poses may be used during evaluation for each (simulated) phantom position. To evaluate the accuracy over a full travel range, multiple virtual shifts of phantom 501 on a couch may be simulated relative to the position where the phantom was placed on the couch.

At 740 in FIG. 7, Monte Carlo simulation(s) may be performed to calibrate each first couch model (*COUCH-q*) numerically using each candidate dataset *(SET-m)* for *m* = 1, ... ,100 and *q* = 1, ...,500. The result of numerical calibration is multiple ($Q \times M$) second couch models. For example, *COUCH'*-1-1 to *COUCH'*-1-100 may be generated for *q* = 1, *COUCH'*-2-1 to *COUCH'*-2-100 for *q* = 2, and so on until *q* = 500. See 750 in FIG. 7.

At 760 in FIG. 7, based on *Q* = 500 first couch models and *V* = 729 validation poses, multiple ($Q \times M$) second couch models may be evaluated. For each validation pose (*POSE-v*), ground truth pose data (*POSE-v-q*) associated with a first couch model and calculated/measured pose data (*POSE'-v-q-m*) associated with a second couch model may be compared to generate deviation data (*DEV-q-m*). For example, *DEV*-1-1 to *DEV*-1-100 may be generated for *q* = 1, *DEV*-2-1 to *DEV*-2-100 for *q* = 2, and so on until *q* = 500. See 770 in FIG. 7.

At 780 in FIG. 7, metric data associated with *SET*-1 to *SET*-100 (*M* = 100) may be generated. For example, *METRIC*-1 may be generated based on {*DEV-q*-1} for *q* = 1, ...,500 and *m* = 1. In another example, *METRIC*-2 may be generated based on {*DEV-q*-2} for *q* = 1, ...,500 and *m* = 2. This may be repeated until *m* = 100. Each metric data (*METRIC-m*) may include mean deviation data, maximum deviation data, deviation range data, or any combination thereof. At 790, a particular candidate dataset (*SET-m\**) may be selected based on the metric data, thereby completing calibration dataset generation process.

(b) Comparison of different dataset sizes

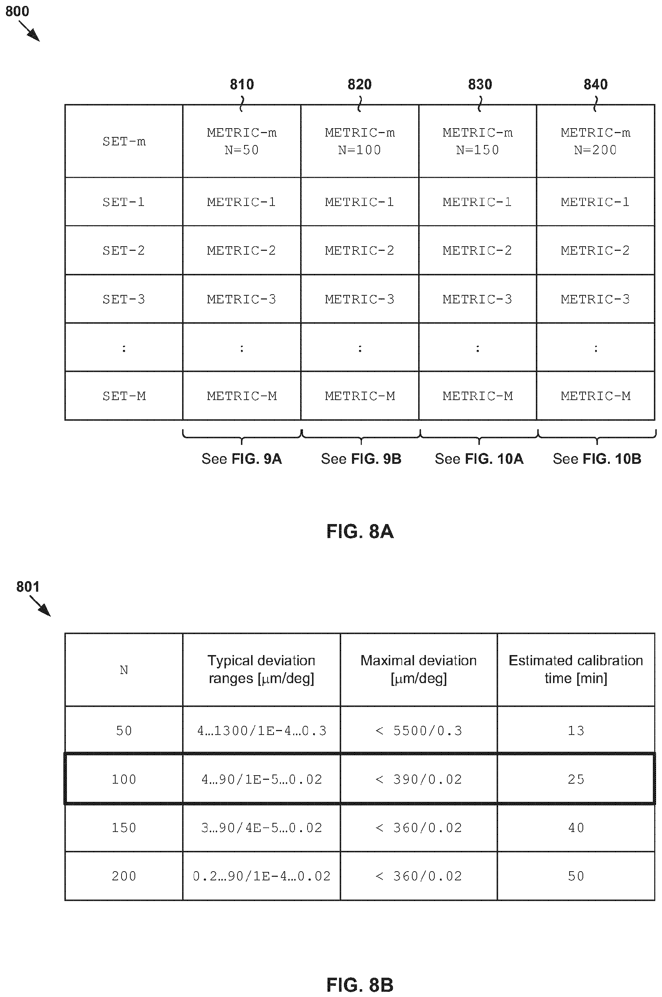

FIG. 8A is first table 800 illustrating example metric data associated with multiple candidate datasets. In practice, Monte Carlo simulations in the example in FIG. 7 may be repeated for different dataset sizes, such as $N = 50$, 100, 150 and 200 to assess possible tradeoffs between calibration accuracy and calibration time. For a particular $M$, a first set of metric data may be generated for $N = 50$ (see 810), a second set for $N = 100$ (see 820), a third set for $N = 150$ (see 830) and a fourth set for $N = 200$ (see 840). In practice, each $N$ generally incurs a different amount of calibration time. For example, one measurement (e.g., using an orthogonal X-ray imaging pair) may be in the order of 15 seconds, resulting in 13 minutes for $N = 50$, 25 minutes for $N = 100$, 38 minutes for $N = 150$ and 50 minutes for $N = 200$ during on-site couch calibration.

FIG. 8B is second table 801 illustrating example metric data 801 associated with different candidate dataset sizes ($N$). Here, deviation data for combined isocentric shifts of 5 cm and rotations of 3 degrees for varying dataset size ($N$) are summarized in second table 801. From the results, first computer system 180 may select $N = 100$ calibration poses per dataset to balance between calibration accuracy and calibration time. The maximum deviation data is below 0.39 mm and 0.02 degree. The estimated calibration time for $N = 100$ poses is around 25 minutes (e.g., 15 seconds per pose). In practice, dataset selection may be performed based on a tradeoff between calibration accuracy and calibration time. For example, $N = 150$ may be selected to achieve higher calibration accuracy, even though more calibration time (e.g., 40 minutes) is required.

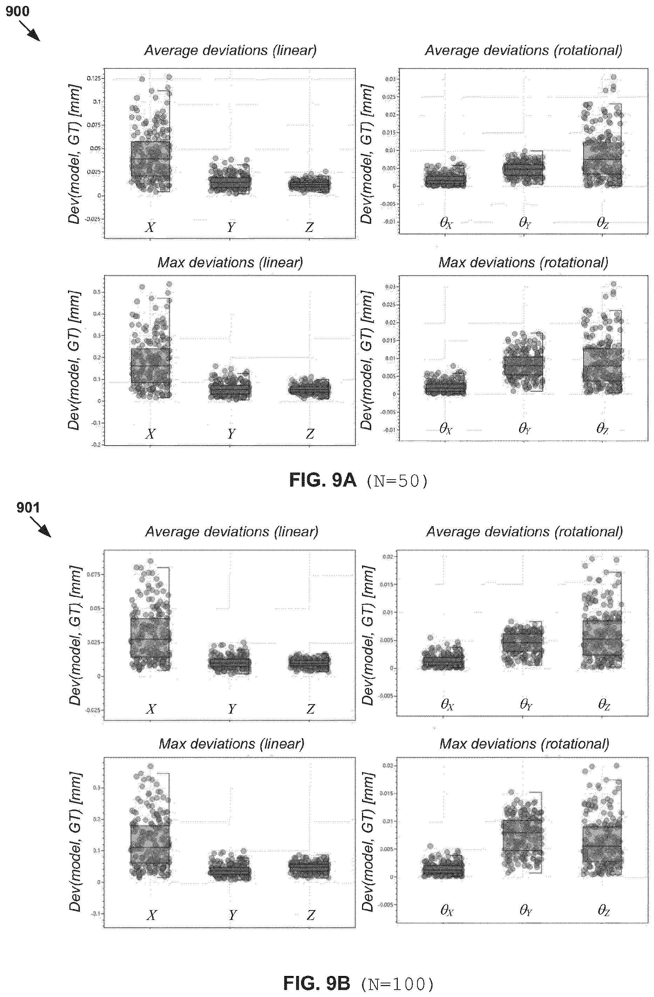

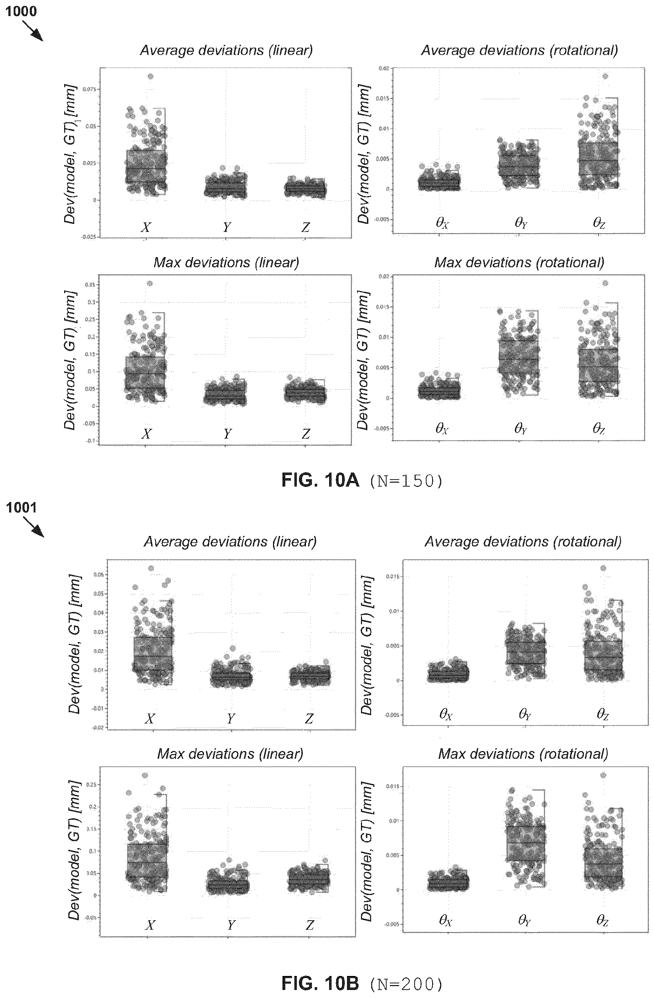

Boxplots for each dataset size ($N$) may be found in FIGs. 9A-B and FIGs. 10A-B. In particular, FIG. 9A are example boxplots 900 displaying average and maximum deviation data for linear and rotational components for a dataset size of $N = 50$ poses. FIG. 9B are example boxplots 901 displaying average and maximum deviation data for linear and rotational components for a dataset size of $N = 100$ poses. FIG. 10A are example boxplots 1000 displaying average and maximum deviation data for linear and rotational components for a dataset size of $N = 150$ poses. Further, FIG. 10B are example boxplots 1001 displaying average and maximum deviation data for linear and rotational components for a dataset size of $N = 200$ poses.

In practice, a couch may be calibrated for one phantom position but needs to be validated for the full travel range. FIGs. 9A-B and FIGs. 10A-B include results for the full travel range of a couch, which may indicate the worst cases. Depending on the desired implementation, evaluation may be split according to the position of phantom 501 on the couch for phantom-position-dependent analysis of calibration accuracy. In practice, it has been observed that the calibration accuracy may improve when calibrating the model at multiple phantom positions relative to the couch. However, this comes at the cost of increasing the calibration time, and requiring user interaction, as the phantom would need to be moved during the calibration process.

Computer system

The above examples can be implemented by hardware (including hardware logic circuitry), software or firmware or a combination thereof. The above examples may be implemented by any suitable computing device, computer system, etc. The computer system may include processor(s), memory unit(s) and physical NIC(s) that may communicate with each other via a communication bus, etc. The computer system may include a non-transitory computer-readable medium having stored thereon instructions or program code that, when executed by the processor, cause the processor to perform processes described herein with reference to the drawings.

The techniques introduced above can be implemented in special-purpose hardwired circuitry, in software and/or firmware in conjunction with programmable circuitry, or in a combination thereof. Special-purpose hardwired circuitry may be in the form of, for example, one or more application-specific integrated circuits (ASICs), programmable logic devices (PLDs), field-programmable gate arrays (FPGAs), and others. The term 'processor' is to be interpreted broadly to include a processing unit, ASIC, logic unit, or programmable gate array etc.

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or any combination thereof.

Those skilled in the art will recognize that some aspects of the embodiments disclosed herein, in whole or in part, can be equivalently implemented in integrated circuits, as one or more computer programs running on one or more computers (e.g., as one or more programs running on one or more computing systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure.

Software to implement the techniques introduced here may be stored on a non-transitory computer-readable storage medium and may be executed by one or more general-purpose or special-purpose programmable microprocessors. A "computer-readable storage medium", as the term is used herein, includes any mechanism that provides (i.e., stores and/or transmits) information in a form accessible by a machine (e.g., a computer, network device, personal digital assistant (PDA), mobile device, manufacturing tool, any device with a set of one or more processors, etc.). A computer-readable storage medium may include recordable/non recordable media (e.g., read-only memory (ROM), random access memory (RAM), magnetic disk or optical storage media, flash memory devices, etc.).

The drawings are only illustrations of an example, wherein the units or procedure shown in the drawings are not necessarily essential for implementing the present disclosure. Those skilled in the art will understand that the units in the device in the examples can be arranged in the device in the examples as described or can be alternatively located in one or more devices different from that in the examples. The units in the examples described can be combined into one module or further divided into a plurality of sub-units.

**Claims**

1.  A method for a computer system to perform a numerical calibration approach for treatment couch calibration, wherein the method comprises:

    obtaining multiple candidate datasets and multiple first couch models, wherein the multiple candidate datasets include at least (a) a first dataset of multiple first calibration poses and (b) a second dataset of multiple second calibration poses;
    based on the multiple candidate datasets and the multiple first couch models, performing numerical calibration to generate multiple second couch models;
    based on the multiple first couch models and the multiple second couch models, determining metric data associated with the multiple candidate datasets, wherein the metric data includes at least (a) first metric data associated with the first dataset and (b) second metric data associated with the second dataset; and
    selecting, from the multiple candidate datasets, the first dataset based on the metric data, wherein the first dataset is selected for use by a controller associated with a treatment couch to cause the treatment couch to move towards each of the multiple first calibration poses during treatment couch calibration.

2.  The method of claim 1, wherein selecting the first dataset comprises:
    selecting the first dataset based on one or more of the following selection criteria: (a) calibration accuracy that is dependent on the multiple first calibration poses in the first dataset and (b) calibration time that is dependent on a size of the first dataset.

3.  The method of claim 1 or claim 2, wherein performing numerical calibration comprises:
    generating a particular second couch model from the multiple second couch models by performing numerical calibration of a particular first couch model from the multiple first couch models based on the multiple first calibration poses of the first dataset;
    and/or:
    wherein generating the particular second couch model comprises:
    determining measured pose data associated with the particular first couch model based on at least one of the following: a kinematic model associated with the particular first couch model and a measurement accuracy offset.

4.  The method of any preceding claim, wherein determining the metric data comprises:

    determining ground truth pose data associated with a particular first couch model from the multiple first couch models based on a particular validation pose;
    determining measured pose data associated with a particular second couch model from the multiple second couch models based on the particular validation pose; and
    based on the ground truth pose data and calculated pose data, determining deviation data associated with (a) the particular first couch model and (b) the particular second couch model that is generated based on the first dataset.

5.  The method of claim 4, wherein determining the metric data comprises:
    determining the first metric data associated with the first dataset based on deviation data associated with (a) multiple first couch models and (b) multiple second couch models that are generated based on the first dataset;
    and/or:
    wherein determining the metric data comprises:
    determining the first metric data that includes one or more of the following: average deviation data, maximum deviation data and deviation data range.

6.  A computer system, comprising:

a processor; and

a non-transitory computer-readable medium having stored thereon instructions that, when executed by the processor, cause the processor to perform the following:

obtain multiple candidate datasets and multiple first couch models, wherein the multiple candidate datasets include at least (a) a first dataset of multiple first calibration poses and (b) a second dataset of multiple second calibration poses;

based on the multiple candidate datasets and the multiple first couch models, perform numerical calibration to generate multiple second couch models;

based on the multiple first couch models and the multiple second couch models, determine metric data associated with the multiple candidate datasets, wherein the metric data includes at least (a) first metric data associated with the first dataset and (b) second metric data associated with the second dataset; and

select, from the multiple candidate datasets, the first dataset based on the metric data, wherein the first dataset is selected for use by a controller associated with a treatment couch to cause the treatment couch to move towards each of the multiple first calibration poses during treatment couch calibration.

7. The computer system of claim 6, wherein the instructions for selecting the first dataset cause the processor to:
select the first dataset based on one or more of the following selection criteria: (a) calibration accuracy that is dependent on the multiple first calibration poses in the first dataset and (b) calibration time that is dependent on a size of the first dataset.

8. The computer system of claim 6 or claim 7, wherein the instructions for performing numerical calibration cause the processor to:
generate a particular second couch model from the multiple second couch models by performing numerical calibration of a particular first couch model from the multiple first couch models based on the multiple first calibration poses of the first dataset;
and/or:
wherein the instructions for generating the particular second couch model cause the processor to:
determine measured pose data associated with the particular first couch model based on at least one of the following: a kinematic model associated with the particular first couch model and a measurement accuracy offset.

9. The computer system of any of claim 6 to claim 8, wherein the instructions for determining the metric data cause the processor to:

determine ground truth pose data associated with a particular first couch model from the multiple first couch models based on a particular validation pose;

determine measured pose data associated with a particular second couch model from the multiple second couch models based on the particular validation pose; and

based on the ground truth pose data and calculated pose data, determine deviation data associated with (a) the particular first couch model and (b) the particular second couch model that is generated based on the first dataset.

10. The computer system of claim 9, wherein the instructions for determining the metric data cause the processor to:
determine the first metric data associated with the first dataset based on deviation data associated with (a) multiple first couch models and (b) multiple second couch models that are generated based on the first dataset;
and/or:
wherein the instructions for determining the metric data cause the processor to:
determine the first metric data that includes one or more of the following: average deviation data, maximum deviation data and deviation data range.

11. A radiation therapy system, comprising:

a treatment couch that requires calibration; and
a couch calibration system to:

obtain a selected dataset of multiple calibration poses that is selected from multiple candidate datasets based on metric data associated with the multiple candidate datasets, wherein (a) numerical calibration is performed based on the multiple candidate datasets and multiple first couch models to generate multiple second couch models, and (b) the metric data is generated based on the multiple first couch models and the multiple second couch models; and

perform couch calibration based on the selected dataset by (a) instructing the treatment couch to move towards each of the multiple calibration poses in the selected dataset, (b) estimating measured pose data associated with the multiple calibration poses, and (c) determining geometric correction data associated with the treatment couch based on the measured pose data.

12. The radiation therapy system of claim 11, further comprising a numerical calibration system to:

obtain the multiple candidate datasets and the multiple first couch models, wherein the multiple candidate datasets include at least (a) a first dataset of multiple first calibration poses and (b) a second dataset of multiple second calibration poses;

based on the multiple candidate datasets and the multiple first couch models, perform numerical calibration to generate the multiple second couch models;

based on the multiple first couch models and the multiple second couch models, determine the metric data that includes at least (a) first metric data associated with the first dataset and (b) second metric data associated with the second dataset; and

based on the metric data, select the first dataset, being the selected dataset, from the multiple candidate datasets.

13. The radiation therapy system of claim 12, wherein the numerical calibration system is to perform numerical calibration by performing the following:

generate a particular second couch model from the multiple second couch models based on (a) a particular first couch model from the multiple first couch models and (b) the multiple first calibration poses of the first dataset; and/or:

wherein the numerical calibration system is to generate the particular second couch model by performing the following:

determine measured pose data based on at least one of the following: a kinematic model associated with the particular first couch model and a measurement accuracy offset.

14. The radiation therapy system of claim 13, wherein the numerical calibration system is to determine the metric data by performing the following:

determine ground truth pose data associated with a particular first couch model from the multiple first couch models based on a particular validation pose;

determine measured pose data associated with a particular second couch model from the multiple second couch models based on the particular validation pose; and

based on the ground truth pose data and calculated pose data, determine deviation data associated with (a) the particular first couch model and (b) the particular second couch model that is generated based on the first dataset.

15. The radiation therapy system of any of claim 11 to claim 14, wherein the couch calibration system is to obtain the selected dataset by performing the following:

obtain the selected dataset that is selected based on one or more of the following selection criteria: (a) calibration accuracy that is dependent on the multiple calibration poses in the selected dataset and (b) calibration time that is dependent on a size of the first dataset.

FIG. 1

FIG. 2

300

Degrees of Freedom (DoF)*

| | | |
|---|---|---|
| X | Lateral | 321 |
| Y | Longitudinal | 322 |
| Z | Vertical | 323 |
| $\theta_X$ | Pitch | 324 |
| $\theta_Y$ | Roll | 325 |
| $\theta_Z$ | Yaw | 326 |

* Examples: 3DoF, 4DoF, 6DoF, etc.

**FIG. 3**

400

## 401 DATASET GENERATION
*E.g., First Computer System*
*180*

**410**
Perform data preparation

> **411**
> Obtain/generate multiple candidate datasets {SET-m}, SET-m={POSE-m-n} for m=1,…,M and n=1,…,N

> **412**
> Obtain/generate multiple first couch models {COUCH-q}, q=1,…,Q

**420**
Perform numerical calibration

> **421**
> Generate second couch models {COUCH'-q-m} based on {COUCH-q} and {SET-m}

**430**
Based on {COUCH-q} and {COUCH'-q-m}, evaluate {SET-m} by determining metric data {METRIC-m}, m=1,…,M

**440**
Based on metric data {METRIC-m}, select candidate dataset SET-m* for use in couch calibration

## 402 ON-SITE CALIBRATION
*E.g., Second Computer System*
*190*

**445**
Dataset of calibration poses:
SET-m*={POSE-m*-n}
and n=1,…,N

**450**
Obtain SET-m*
={POSE-m*-n}

For each n

**460**
Instruct uncalibrated couch (on which phantom is placed) to move towards POSE-m*-n

**470**
Obtain imaging data from imager(s) and/or laser tracking data from laser tracker(s)

**480**
Determine measured pose data (POSE'-m*-n) reached by couch based on imaging/tracking data

n<N

n=N

**490**
Determine geometric correction data (p) based on at least {POSE'-m*-n}

**495**
Adjust couch based on p (e.g., manually or programmatically)

## FIG. 4

**500**

Phantom
**501**

Isocenter
**502**

**303**

**131**

$J_{\theta X}$

$c_1$=L(Roll →
Table Base Point)
**551***

$J_Y$ $J_{\theta Y}$

$c_2$=H(IEC → Lat)
**552***

$J_Z$

**302**

$J_{\theta Z}$

$c_3$=L(IEC → Lat)
**553***

$J_X$ **301**

* **550** Couch-dependent mechanical
parameter data
$c=[c_1, c_2, c_3, ...]$

**510**
Joint parameter data
(JOINT)

| |
|---|
| $J_X$ |
| $J_Y$ |
| $J_Z$ |
| $J_{\theta X}$ |
| $J_{\theta Y}$ |
| $J_{\theta Z}$ |

*Couch joint space*

**530**
Forward Kinematic Model
JOINT=FKM(POSE,c,p)**

**540**
Inverse Kinematic Model
POSE=IKM(JOINT,c,p)**

**520**
Pose data
(POSE)

| |
|---|
| $X$ |
| $Y$ |
| $Z$ |
| $\theta_X$ |
| $\theta_Y$ |
| $\theta_Z$ |

*IEC fixed coordinate
system*

**Geometric correction data
$p=[p_1, p_2, p_3, ...]$

**FIG. 5**

**600**

### 605
Perform
data preparation
based on $(M, N, Q, V)$

### 610
Generate $M$ candidate datasets of random calibration poses:
$\{SET-m\}$ where $SET-m=\{POSE-m-n\}$ for $m=1,\ldots,M$ and
$POSE-m-n=(X, Y, Z, \theta_X, \theta_Y, \theta_Z)$ for $n=1,\ldots,N$

### 620
Generate $Q$ first couch models: $\{COUCH-q\}$, where
each $COUCH-q$ (ground truth) is generated based on random
offset $(\delta)$ to simulate measurement accuracy for $q=1,\ldots,Q$

### 630
Generate dataset of $V$ validation poses:
$\{POSE-v\}$ where each $POSE-v$ is a pose selected for
evaluating couch models

### 640
Perform
numerical calibration
(optimization)

### 650
Perform numerical calibration of $COUCH-q$ based on
$SET-m=\{POSE-m-n\}$, kinematic model $(KM)$ and random offset
$(\delta)$ to simulate measurement inaccuracy

```
for q=1,…,Q
    for m=1,…,M
        for n=1,…,N
            #calculated/measured pose data
651         POSE'q-m-n=KM(COUCH-q)+δ
652         DEV-q-m-n=diff(POSE-m-n,POSE'-q-m-n)
        end
        COUCH'-q-m=OPTIMIZE({DEV-q-m-n})    653
    end
end
```

### 660
Perform
evaluation

### 670
Evaluate $COUCH-q$ and $COUCH'-q-m$ using $\{POSE-v\}$
to determine metric data $(METRIC-m)$

```
for m=1,…,M
    for q=1,…,Q
        for v=1,…,V
            #groundTruth pose data
671         POSE-v-q=f(POSE-v,COUCH-q)
            #calculated/measured pose data
672         POSE'-v-q-m=f(POSE-v,COUCH'-q-m)
673         DEV-q-m=diff(POSE-v-q,POSE'-v-q-m)
        end
    end
    METRIC-m=f({DEV-q-m})    680
end
```

### 690
Calibration dataset
selection

### 695
Select $SET-m^*$ from $\{SET-m\}$ based on $\{METRIC-m\}$ and one
or more selection criteria, where $m^* \in [1,\ldots,M]$

## FIG. 6

700

**710**
{SET-m}, M=100 and N=50

{COUCH-q},
Q=500

{POSE-v},
V=3^6=729

**711**
SET-1=
{POSE-1-n}
n=1,...,N

**712**
SET-2=
{POSE-2-n}
n=1,...,N

**713**
SET-100=
{POSE-100-n}
n=1,...,N

**720**
COUCH-1
⋮
COUCH-500

**730**
POSE-1
⋮
POSE-729

**740**
Monte Carlo simulation (optimization) based on
{SET-m}, {COUCH-q}, kinematic model and
measurement accuracy offset (δ)

**750** Second Couch Models {COUCH'-q-m}

q=1:     COUCH'-1-1 ... COUCH'-1-100
q=2:     COUCH'-2-1 ... COUCH'-2-100
  ⋮
q=100: COUCH'-500-1 ... COUCH'-500-100

**760**
Evaluation of {COUCH'-q-m}:
DEV-q-m=diff(POSE-v-q, POSE'-v-q-m)
for v=1,...,V, q=1,...,Q and m=1,...,M

Q=500

V=729

**770** Deviation Data {DEV-q-m}

q=1:     DEV-1-1 ... DEV-1-100
q=2:     DEV-2-1 ... DEV-2-100
  ⋮
q=100: DEV-500-1 ... DEV-500-100

**780** Metric Data {METRIC-m}

m=1:     METRIC-1=f({DEV-q-1})
m=2:     METRIC-2=f({DEV-q-2})
  ⋮
m=100: METRIC-100=f({DEV-q-100})

*_Examples_
METRIC-m
=mean({DEV-q-m})
=max({DEV-q-m})

**790**
Dataset generation by selecting SET-m* based on
comparison of {METRIC-m}, where m* ∈ [1,...,M]

FIG. 7

800

| | 810 | 820 | 830 | 840 |
|---|---|---|---|---|
| SET-m | METRIC-m N=50 | METRIC-m N=100 | METRIC-m N=150 | METRIC-m N=200 |
| SET-1 | METRIC-1 | METRIC-1 | METRIC-1 | METRIC-1 |
| SET-2 | METRIC-2 | METRIC-2 | METRIC-2 | METRIC-2 |
| SET-3 | METRIC-3 | METRIC-3 | METRIC-3 | METRIC-3 |
| : | : | : | : | : |
| SET-M | METRIC-M | METRIC-M | METRIC-M | METRIC-M |

See **FIG. 9A**　　See **FIG. 9B**　　See **FIG. 10A**　　See **FIG. 10B**

**FIG. 8A**

801

| N | Typical deviation ranges [μm/deg] | Maximal deviation [μm/deg] | Estimated calibration time [min] |
|---|---|---|---|
| 50 | 4...1300/1E-4...0.3 | < 5500/0.3 | 13 |
| 100 | 4...90/1E-5...0.02 | < 390/0.02 | 25 |
| 150 | 3...90/4E-5...0.02 | < 360/0.02 | 40 |
| 200 | 0.2...90/1E-4...0.02 | < 360/0.02 | 50 |

**FIG. 8B**

900

FIG. 9A (N=50)

901

FIG. 9B (N=100)

1000

Average deviations (linear)

Average deviations (rotational)

Max deviations (linear)

Max deviations (rotational)

FIG. 10A (N=150)

1001

Average deviations (linear)

Average deviations (rotational)

Max deviations (linear)

Max deviations (rotational)

FIG. 10B (N=200)

Europäisches
Patentamt

European
Patent Office

Office européen
des brevets

## EUROPEAN SEARCH REPORT

**Application Number**

EP 25 19 6762

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2020/061392 A1 (FILIBERTI RETO W [CH] ET AL) 27 February 2020 (2020-02-27) * paragraphs [0037], [0044] - [0052]; figure 3; tables 1-4 * ----- | 1-15 | INV. A61N5/10 |
| A | WO 2019/056134 A1 (UNIV DALHOUSIE [CA]) 28 March 2019 (2019-03-28) * page 20, line 12 - page 27, line 25; figures 2A-2C * ----- | 1-15 | |
| A | US 2016/174930 A1 (BRAUN CHRISTOPH [DE] ET AL) 23 June 2016 (2016-06-23) * paragraphs [0046] - [0047] * ----- | 1-15 | |

**TECHNICAL FIELDS SEARCHED (IPC)**

A61N

The present search report has been drawn up for all claims

| Place of search | Date of completion of the search | Examiner |
|---|---|---|
| Munich | 30 November 2025 | Büchler Costa, Joana |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...............................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 19 6762

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2025

| Patent document cited in search report | Publication date | Patent family member(s) | Publication date |
|---|---|---|---|
| US 2020061392 A1 | 27-02-2020 | US 2016023019 A1 | 28-01-2016 |
| | | US 2017128750 A1 | 11-05-2017 |
| | | US 2020061392 A1 | 27-02-2020 |
| | | US 2022219017 A1 | 14-07-2022 |
| WO 2019056134 A1 | 28-03-2019 | NONE | |
| US 2016174930 A1 | 23-06-2016 | CN 105708485 A | 29-06-2016 |
| | | DE 102014226756 A1 | 03-03-2016 |
| | | KR 20160076487 A | 30-06-2016 |
| | | US 2016174930 A1 | 23-06-2016 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 09679 A

- EP 18809573 A

**Non-patent literature cited in the description**

- **W. H. PRESS** ; **S. H. TEUKOLSKY** ; **W. T. VETTERLING** ; **B. P. FLANNERY**. *Numerical Recipes: The Art of Scientific Computing*